# EUROPEAN PATENT APPLICATION

(11) **EP 2 236 160 A2**
(43) Date of publication of application: **06.10.2010**
(21) Application number: 10158257.5
(22) Date of filing: 29.03.2010
(51) Int. Cl.: A61K 47/30, A61K 9/20, A61K 31/437

(54) **Modified release dimebolin formulations**

(30) Priority: 25.02.2010 TR 201001416; 31.03.2009 TR 200902500
(71) Applicant: Sanovel Ilac Sanayi ve Ticaret A.S., 34398 Istanbul (TR)
(72) Inventor: Toksöz, Ahmet, 34398, Istanbul (TR); Cifter, Ümit, 34398, Istanbul (TR); Turkyilmaz, Ali, 34398, Istanbul (TR)
(74) Representative: Sevinç, Erkan

(57) **Abstract**

According to the present invention a modified release formulation comprising dimebolin or a pharmaceutically acceptable salt thereof and a rate controlling polymer which is selected from the group comprising polyvinylpyrrolidine, hydroxypropyl cellulose, hydroxypropylmethyl cellulose, hydroxymethyl cellulose, hydroxyethyl cellulose, hydroxypropylethyl cellulose, microcrystalline cellulose, methyl cellulose, carboxyethyl cellulose, carbomer, sodium carboxy methyl cellulose, cellulose acetatebutyrate, vinyl acetate/crotonic acid copolymers, maleic anhydride/methyl vinyl ether copolymers, copolymer of acrylic and methacrylic acid, ethyacrylic acid, shellac, waxes, zein, ethyl cellulose and derivatives and mixtures, together with one or more pharmaceutically acceptable excipients wherein the maximum 30% of total amount of dimebolin or a pharmaceutically acceptable salt is released in 2 hours and 40-65% in 6 hours and at least 85% between a period of 16 to 24 hours is provided.

## Description

### Technical Aspect

The present invention is related to a modified release formulation comprising dimebolin or a pharmaceutically acceptable salt thereof and a rate controlling polymer which is selected from the group comprising polyvinylpyrrolidine, hydroxypropyl cellulose, hydroxypropylmethyl cellulose, hydroxymethyl cellulose, hydroxyethyl cellulose, hydroxypropylethyl cellulose, microcrystalline cellulose, methyl cellulose, carboxyethyl cellulose, carbomer, sodium carboxy methyl cellulose, cellulose acetatebutyrate, vinyl acetate/crotonic acid copolymers, maleic anhydride/methyl vinyl ether copolymers, copolymer of acrylic and methacrylic acid, ethyacrylic acid, shellac, waxes, zein, ethyl cellulose and derivatives and mixtures, together with one or more pharmaceutically acceptable excipients wherein the maximum 30% of total amount of dimebolin or a pharmaceutically acceptable salt is released in 2 hours and 40-65% in 6 hours and at least 85% between a period of 16 to 24 hours.

### Background of the Invention

Dimebolin is an antiallergic agent and N-methyl d-aspartat receptor antagonist (NMDA). Its chemical name is 2,8-dimethyl-5-(2-(6-methyl-3-pyridyl)ethyl)-2,3,4,5-tetrahydro-1H-pyrido[4,3-b]indole and its chemical structure is shown in the Formula I.

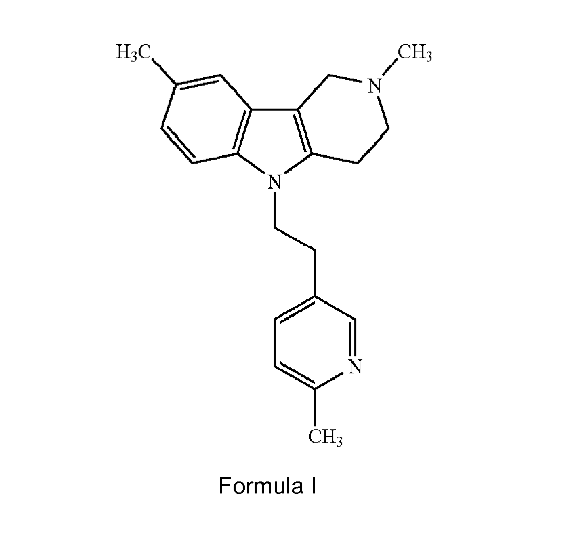

Dimebolin has been used as an antiallergic agent (Inventor's Certificate No. 1138164, IP Class A61K 31/47,5, C07 D 209/52, published on Feb. 7, 1985) in Russia for over 20 years. It has hereto been administered orally in the form of tablets containing a dose from 2.5 mg to 20 mg. The usual prescription is 1 tablet in three times a day.

As described in U.S. Patent No. 6,187,785 and 7,071,206, hydrogenated pyrido[4,3-b]indole derivatives, such as dimebolin, have NMDA receptor antagonist properties, which make them useful for treating neurodegenerative diseases, such as Alzheimer's disease. As described in WO 2005/055951, hydrogenated pyrido[4,3-b]indole derivatives, such as dimebolin, are useful as human or veterinary geroprotectors. They work by delaying the onset and/or development of an age-associated or related manifestation and/or pathology or condition, including disturbance in skin-hair integument, vision disturbance and weight loss. U.S. Provisional Patent Application No. 60/723,403, filed October 4, 2006, and U.S. Patent Application No. 11/543,529, filed October 4, 2006, disclose hydrogenated pyrido[4,3- b] indole derivatives, such as dimebolin, as neuroprotectors for use in treating and/or preventing and/or slowing the progression or onset and/or development of Huntington's disease. RU application which is filed January 25, 2006 with title of "Agent for Treatment of Schizophrenia Based on Hydrogenated Pyrido [4,3 -b] indoles (Variations), a Pharmacological Agent Based on it, and a Method of Using it" is another publication in this field.

It is desirable in the treatment of a number of diseases, to provide the active pharmaceutical ingredient in a modified release form. Desirably the modified release formulation provides a generally uniform and constant rate of release over a modified period of time which achieves desired plasma level of the active ingredient without the need for frequent administration of the medicament. Accordingly, the terms "modified-release", controlled-release", "prolonged-release", "extended-release", and "sustained-release" are used interchangeably herein.

Because of several different reasons it is difficult to develop modified release formulations of highly soluble pharmaceuticals although there are many modified release formulations formulated with rate controlling polymers. First of all, modified release formulations of highly soluble medicaments can be prone to "dose dumping" in which the release of the active ingredient is delayed but once the release begins the medicament is released very fast. It is also hard to achieve the desired dissolution profiles in other words the control of the release rate is difficult. Therefore, fluctuation of the active ingredient concentration in the plasma may occur which may lead to toxicity. Also, diurnal variation of the active ingredient in plasma is also possible.

Accordingly, a need rises for modified release formulations of soluble medicaments such as dimebolin or pharmaceutically acceptable salt, which overcomes the above described problems and which further provide the advantageous property of allowing the active medicament to be administered less frequently, e.g. once-a-day or twice-a-day, while achieving comparable plasma levels to immediate release form.

### Description of the invention

According to the present invention a modified release formulation comprising dimebolin or a pharmaceutically acceptable salt thereof and a rate controlling polymer which is selected from the group comprising polyvinylpyrrolidine, hydroxypropyl cellulose, hydroxypropylmethyl cellulose, hydroxymethyl cellulose, hydroxyethyl cellulose, hydroxypropylethyl cellulose, microcrystalline cellulose, methyl cellulose, carboxyethyl cellulose, carbomer, sodium carboxy methyl cellulose, cellulose acetatebutyrate, vinyl acetate/crotonic acid copolymers, maleic anhydride/methyl vinyl ether copolymers, copolymer of acrylic and methacrylic acid, ethyacrylic acid, shellac, waxes, zein, ethyl cellulose and derivatives and mixtures, together with one or more pharmaceutically acceptable excipients wherein the maximum 30% of total amount of dimebolin or a pharmaceutically acceptable salt is released in 2 hours and 40-65% in 6 hours and at least 85% between a period of 16 to 24 hours is provided.

In one embodiment the amount of dimebolin or a pharmaceutically acceptable salt thereof is present in 1 to 60% by weight of total formulation, preferably 25 to 60%, the more preferably 25 to 35%. Dimebolin is preferably in the form of an HCl salt.

In a further aspect, the present invention relates to the modified release formulations comprising dimebolin or a pharmaceutically acceptable salt thereof, in an amount of 1 mg to 420mg. The modified release formulation is to be administered once-a-day or twice-a-day.

It has surprisingly found that in this modified release formulation having a weight ratio of dimebolin or a pharmaceutically acceptable salt to rate controlling polymer between the ranges of 10:1 to 1:10 (w/w) has a synergistic effect over the dissolution rate.

The formulations of the invention comprise one or more excipients. Such excipients include diluents, fillers, disintegrants, binders, glidants, preservatives and lubricants.

Suitable diluents and fillers may include but not limited to lactose, microcrystalline cellulose, starch, mannitol, glucose, and the like and mixtures thereof; preferably starch.

Suitable disintegrants may include but not limited to microcrystalline cellulose, sodium starch glycollate, croscarmellose sodium, crospovidone, starch and the like and mixtures thereof; preferably microcrystalline cellulose.

Suitable binders may include but not limited to povidone, sucrose, polyethylene glycol and the like and mixtures thereof, preferably povidone.

Suitable glidants may include but not limited to colloidal silicon dioxide, talc, aluminium silicate and the like and mixtures thereof, preferably colloidal silicon dioxide.

Suitable preservatives may include but not limited to methyl paraben, propyl paraben, sodium benzoate, citric acid and benzoic acid and the like and mixtures thereof, preferably citric acid.

Suitable lubricants may include but not limited to magnesium strearate, calcium stearate, sodium stearyl fumarate, stearic acid and the like and mixtures thereof, preferably magnesium strearate and stearic acid.

These modified release pharmaceutical formulations are administrated by oral, parenteral, intramuscular or topical route. The modified release pharmaceutical formulations of the invention include tablet, capsule, sachet, microcapsules, minitablet, multilayer and multicoated tablet, pellet, injectable preparation, suspension, syrup, gel, cream or ointment which can be formulated in accordance with methods that are standard in the art.

The term rate controlling polymer includes substance such as polyvinylpyrrolidine, hydroxypropyl cellulose, hydroxypropylmethyl cellulose, hydroxymethyl cellulose, hydroxyethyl cellulose, hydroxypropylethyl cellulose, microcrystalline cellulose, methyl cellulose, carboxyethyl cellulose, carbomer, sodium carboxy methyl cellulose, cellulose acetatebutyrate, vinyl acetate/crotonic acid copolymers, maleic anhydride/methyl vinyl ether copolymers, copolymer of acrylic and methacrylic acid, ethyacrylic acid, shellac, waxes, zein, ethyl cellulose and derivatives and mixtures. The rate controlling polymer is preferably hydroxypropyl methylcellulose.

The amount of rate controlling polymer, hydroxypropyl methylcellulose is preferably adjusted to provide a release of maximum 30% of the total amount of dimebolin or a pharmaceutically acceptable salt in 2 hours and 40-65% in 6 hours and at least 85% between a period of 16 to 24 hours.

The rate controlling polymer, preferably hydroxypropyl methylcellulose is present 3 to 80 % by weight of total formulation, particularly 20 to 40%.

The rate controlling polymer is preferably ethyl cellulose. The amount of ethyl cellulose is preferably selected to provide a release of maximum 30% of the total amount of dimebolin or a pharmaceutically acceptable salt in 2 hours and 40-65% in 6 hours and at least 85% between a period of 16 to 24 hours.

The rate controlling polymer, preferably ethyl cellulose is present 0.5 to 40 % by weight of total formulation, particularly 1 to 20%.

The formulation contains one or more pharmaceutically acceptable excipients which are selected from the group comprising (a) 4 to 60% by weight of starch, (b) 4 to 60 % by weight of microcrystalline cellulose, (c) 0.5 to 10% by weight of povidone, (d) 0.5 to 10% by weight of colloidal silicon dioxide and (e) 0.1 to 5% by weight of magnesium stearate.

The formulations of the present invention may be prepared by conventional technology well known to those skilled in the art such as wet granulation, direct compression, dry granulation and the like. Thus, for instance, dimebolin, or a pharmaceutically acceptable salt thereof, a rate controlling polymer and other excipients are mixed together to form the modified release formulations. The prepared mixture is filled into capsules or compressed to form tablets.

The manufacturing process is preferably performed by mixing the components, wet granulating the mixed components; the mixed components, drying the mixture, milling the dried mixture, blending the mixture with a lubricant(s) and compressing the blended mixture to form tablets or filling the blended mixture into capsules.

A preferred process for preparing the formulations of the invention comprises the following steps:
(a) mixing dimebolin, or a pharmaceutically acceptable salt thereof and a rate controlling polymer and other excipients;
(b) wet granulating the mixing components;
(c) drying the mixture;
(d) milling the dried mixture;
(e) blending the mixture with a lubricant; and
(f) compressing the blended mixture to form tablets, and optionally coating said tablets.

The dosage forms may be coated with one or more coatings as is well known in the art such as, for example, shellac, zein, hydroxyproply cellulose, hydroxypropyl methylcellulose, ethly cellulose, polymethacrylates, polyvinyl acetate phthalate, cellulose acetate phthalate, triacetin, dibutyl sebacate, polyethylene glycol, titanium dioxide, and the like and mixtures thereof.

The modified release pharmaceutical formulation is preferably enteric coated.

Therefore, further aspects of the present invention concern the use of pharmaceutical formulations comprising dimebolin or a pharmaceutically acceptable salt thereof, together with one or more pharmaceutically acceptable excipients for the manufacture of a medicament for the treatment of Alzheimer disease, neuropathic pain, chronic visceral pain, chronic inflammatory pain, headache, antihistaminic disease in a warm-blooded animal.

Another preferred embodiment of this invention is the formulation which is in the form of a tablet comprising:
(a) a core comprising the dimebolin or a pharmaceutically acceptable salt thereof and pharmaceutically acceptable excipients,
(b) a coating layer comprising a rate controlling polymer.

## Claims

1. A modified release formulation comprising dimebolin or a pharmaceutically acceptable salt thereof and a rate controlling polymer which is selected from the group comprising polyvinylpyrrolidine, hydroxypropyl cellulose, hydroxypropylmethyl cellulose, hydroxymethyl cellulose, hydroxyethyl cellulose, hydroxypropylethyl cellulose, microcrystalline cellulose, methyl cellulose, carboxyethyl cellulose, carbomer, sodium carboxy methyl cellulose, cellulose acetatebutyrate, vinyl acetate/crotonic acid copolymers, maleic anhydride/methyl vinyl ether copolymers, copolymer of acrylic and methacrylic acid, ethyacrylic acid, shellac, waxes, zein, ethyl cellulose and derivatives and mixtures, together with one or more pharmaceutically acceptable excipients wherein the maximum 30% of total amount of dimebolin or a pharmaceutically acceptable salt is released in 2 hours and 40-65% in 6 hours and at least 85% between a period of 16 to 24 hours.

2. The modified release formulation according to claim 1 wherein dimebolin or a pharmaceutically acceptable salt thereof is present in 1 to 60% by weight of total formulation, preferably 25 to 60% by weight of total formulation.

3. The modified release formulation according to any proceeding claims, wherein the formulation is to be administered once-a-day or twice-a-day.

4. The modified release formulation according to claim 1-3, wherein the weight ratio of dimebolin or a pharmaceutically acceptable salt to rate controlling polymer is in the range of between 10:1 to 1:10 (w/w).

5. The modified release formulation according to claims 1-4 wherein the one or more pharmaceutically acceptable excipients are selected from the group comprising diluents, fillers, disintegrants, binders, glidants, preservatives and lubricants.

6. The modified release formulation according to claim 1-5 wherein the rate controlling polymer is hydroxypropyl methylcellulose.

7. The modified release formulation according to claim 6 wherein the amount of hydroxypropyl methylcellulose is 3 to 80% by weight of total formulation.

8. The modified release formulation according to claim 1-5 wherein the rate controlling polymer is ethyl cellulose.

9. The modified release formulation according to claim 8 wherein the amount of ethyl cellulose is 0.5 to 40% by weight of total formulation.

10. The modified release formulation according to any preceding claim wherein the one or more pharmaceutically acceptable excipients which are selected from the group comprising (a) 4 to 60% by weight of starch, (b) 4 to 60 % by weight of microcrystalline cellulose, (c) 0.5 to 10% by weight of povidone, (d) 0.5 to 10% by weight of colloidal silicon dioxide and (e) 0.1 to 5% by weight of magnesium stearate.

11. The modified release formulation according to any preceding claim, wherein the formulation is in the form of a tablet, capsule, sachet, microcapsule, minitablet, multilayer and multicoated tablet, pellet, injectable preparation, suspension, syrup, gel, cream or ointment.

12. The modified release formulation according to anyone of claims 1- 10 further comprising the coating layer.

13. The modified release formulation according to claim 12 wherein the coating layer is preferably enteric coating.

14. The modified release formulation according to 1-10 and 12-13, in the form of a tablet comprising:
(a) a core comprising the dimebolin or a pharmaceutically acceptable salt thereof and pharmaceutically acceptable excipients,
(b) a coating layer comprising a rate controlling polymer.

15. The process for preparing modified release formulation according to 1-10 and 12-14 which comprises
(a) mixing dimebolin, or a pharmaceutically acceptable salt thereof and a rate controlling polymer and other excipients;
(b) wet granulating the mixed components;
(c) drying the mixture;
(d) milling the dried mixture;
(e) blending the mixture with a lubricant; and
(f) compressing the blended mixture to form tablets, and optionally coating said tablets.
